Europäisches Patentamt

European Patent Office

Office européen des brevets

(19)

(11) Veröffentlichungsnummer: **0 213 287**
**B1**

(12) EUROPÄISCHE PATENTSCHRIFT

(45) Veröffentlichungstag der Patentschrift:
04.04.90

(51) Int. Cl.⁴: **A61F 2/34**

(21) Anmeldenummer: **86107476.3**

(22) Anmeldetag: **02.06.86**

(54) **Zementfrei verankerbare Hüftgelenkspfanne.**

(30) Priorität: **15.07.85  CH 3066/85**

(43) Veröffentlichungstag der Anmeldung:
**11.03.87 Patentblatt 87/11**

(45) Bekanntmachung des Hinweises auf die Patenterteilung:
**04.04.90 Patentblatt 90/14**

(84) Benannte Vertragsstaaten:
**AT DE FR GB IT**

(56) Entgegenhaltungen:
**DE-A- 3 406 357**
**FR-A- 2 516 377**

(73) Patentinhaber: **GEBRÜDER SULZER**
**AKTIENGESELLSCHAFT, Zürcherstrasse 9,**
**CH-8401 Winterthur(CH)**

(72) Erfinder: **Gartenmann, Walter, Dr. med., Zürichstrasse 1,**
**CH-8610 Uster 77(CH)**

(74) Vertreter: **Sparing Röhl Henseler Patentanwälte**
**European Patent Attorneys, Rethelstrasse 123,**
**D-4000 Düsseldorf 1(DE)**

## Beschreibung

Die Erfindung betrifft eine zementfrei verankerbare Hüftgelenkpfanne mit mindestens annähernd halbkugelförmigem Pfannenkörper, dessen äußere Oberfläche im Bereich zwischen 15° und 50° "geographischer Breite" mit zwei Reihen zirkularer Rippen bestückt ist, wobei aus der äußeren Oberfläche ein parallel zur Achse des Pfannenkörpers verlaufender Verankerungszapfen herausragt, und wobei ferner für äquatorparallele Durchmesser jeder Rippe gilt, daß der Außendurchmesser der oberen Kante kleiner ist als der Innendurchmesser der Unterkante.

Bei zementfreier Verankerung von Implantaten ist die sogenannte Primärfixation eines der wichtigsten Probleme. Darunter versteht man bekanntlich die Verankerung des Implantates unmittelbar nach dem Einsetzen bis zu einer endgültigen Fixierung durch an- oder einwachsendes Gewebe. Für die Primärfixation von Hüftgelenkspfannen wird dabei häufig ein Klemmsitz angewandt, bei dem die Pfanne in den Beckenknochen, beispielsweise mit Hilfe von einem oder mehreren aus ihrer Außenfläche hervorragenden Zapfen und/oder mit Hilfe von Teilbereichen ihrer Außenfläche, die vorzugsweise nahe dem äquatorialen Rand liegen, eingeklemmt werden (DE-A 3 341 724 und DE-A 3 341 723).

Es hat sich nun gezeigt, daß – besonders bei Konstruktionen, bei denen die Klemmung mindestens teilweise unmittelbar über dem Pfannenkörper erfolgt – Deformationen der eigentlichen Pfannenschale auftreten, die zu einem Einklemmen des Gelenkkopfes der zugehörigen Femurkopfprothese führen können.

Man hat daher schon versucht, den Klemmsitz "zu entschärfen"; hierfür ist eine Hüftgelenkspfanne der eingangs genannten Art aus der DE-A 3 406 357 bekannt. Diese bekannte Konstruktion ist im Bereich mittlerer "geographischer Breiten" auf der äußeren Oberfläche des Pfannenkörpers, die gleichzeitig die Kontaktfläche zum Knochen bildet, mit zwei zirkularen Rippen versehen; die Rippen rasten bei der Implantation der Pfanne nach Art eines Schnappverschlusses in Ringnuten ein, die zusätzlich zur Außenform des Pfannenkörpers in den Beckenknochen gefräst worden sind.

Um das Einsetzen der Rippen in die Ringnuten zu erleichtern, sind die äquatorparallelen Außendurchmesser der einzelnen Rippen so aufeinander abgestimmt, daß der Außendurchmesser ihrer Oberkante kleiner ist als der Innendurchmesser ihrer Unterkante. Dadurch wird der Klemmsitz zwar aufgeweitet und entlastet; in der Praxis hat sich jedoch gezeigt, daß das Einsetzen der vorstehend beschriebenen Pfanne in die in den Knochen gefräste Ringnut Schwierigkeiten bereitet, da der Knochen beim Einsetzen relativ stark aufgespreizt werden muß, ehe die Rippen in die Ringnuten "einschnappen". Dieses Aufspreizen führt leicht zu Beschädigungen des "Knochenbettes", insbesondere an den Rändern der Ringnuten, oder zu Schädigungen der "Einschnappkanten" der Rippen, oder die fixierende Wirkung des Schnappverschlusses bleibt unzureichend.

Aufgabe der vorliegenden Erfindung ist es, die vorstehend beschriebene Hüftgelenkspfanne so zu verbessern, daß die geschilderten Unzulänglichkeiten beseitigt sind; mit anderen Worten sollen Anordnung und Abmessungen der Rippen so ausgebildet werden, daß ein fester Sitz der Pfanne nach dem Einrasten der Rippen in die Ringnuten gewährleistet ist, ohne daß der Knochen oder der Pfannenkörper beim Einsetzen beschädigt werden.

Diese Aufgabe wird mit der Erfindung dadurch gelöst, daß für äquatorparallele Durchmesser jeder Rippe gilt, daß der Außendurchmesser der Unterkante der äquatorferneren Rippen kleiner ist als der Innendurchmesser der Oberkante der äquatornäheren Rippe.

Die erfindungsgemässe Ausbildung der Rippen erlaubt, die Pfanne ohne Knochenberührung in die Operationsöffnung soweit einzusetzen, dass jede der beiden Rippen erst unmittelbar vor der ihr zugehörigen Ringnut an den Knochen ansteht. Es bedarf daher nur noch einer einmaligen Verformung des Pfannenkörpers, um ein "Einschnappen" zu bewirken.

Der in Achsrichtung der Pfanne verlaufende Verankerungszapfen dient dabei im Zusammenwirken mit der für ihn in den Knochen eingebrachten Bohrung als Führung für das Einpressen des Pfannenkörpers in den Schnappverschluss, das in Richtung des Zapfens erfolgt; der Zapfen hat zusätzlich die Aufgabe einer Rotationssicherung.

Weiterhin kann bei der neuen Pfanne, ebenso wie bei der bisherigen Konstruktion, an der Basis des Pfannenkörpers über einen bestimmten Winkelbereich des Umfangs ein hervorstehender Rand vorgesehen sein. Dieser Rand kann zur Verringerung seiner Masse Ausnehmungen aufweisen, so dass einzelne vorstehende Lappen entstehen. Der Rand ist darüberhinaus in seiner Lage relativ zur durch den Zapfen eindeutig festgelegten Einbaustellung der Pfanne derart angeordnet, dass er eine zusätzliche Abstützung der Pfanne in Richtung der Hauptbelastungen bewirkt und weiterhin eventuell auf die Pfanne ausgeübte Kippmomente auffängt; denn wenn die vollen Biegemomente auf die Rippen und die zugehörigen Ringnutbegrenzungen im Knochen wirken, besteht die Gefahr, dass der Knochen im Bereich der Ringnuten ausreisst.

Im folgenden wird die Erfindung anhand eines Ausführungsbeispiels im Zusammenhang mit der Zeichnung näher erläutert.

Fig. 1 ist ein Schnitt I-I von Fig. 2 und zeigt schematisch die neue Hüftgelenkspfanne in den Beckenknochen eingesetzt;

Fig. 2 stellt in einer Ansicht von Fig. 1 von rechts eine Aufsicht auf den Scheitelbereich der neuen Pfanne dar;

Fig. 3 ist in gegenüber Fig. 1 und 2 stark vergrössertem Massstab ein Ausschnitt aus Fig. 1, der den Bereich der Rippen und der Ringnuten wiedergibt.

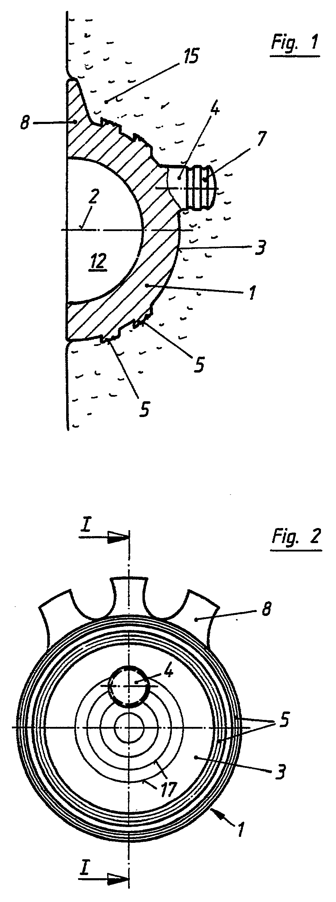

Die Aussenfläche 3 des Pfannenkörpers 1 (Fig. 1) ist im wesentlichen halbkugelförmig, wobei die Kugelschale auf der einen Seite von einem Verankerungszapfen 4 durchbrochen wird. In diesem sind zwei Rillen 7 vorgesehen, in die Knochengewebe einwachsen kann. Der Pfannenkörper 1 und die eigentliche Pfannenschale 12, die den nicht gezeigten Gelenkkopf der Femurkopfprothese aufnimmt, sind rotationssymmetrisch zur Achse 2 des Pfannenkörpers 1 ausgebildet.

Weiterhin ragen aus der Aussenfläche 3 zwei zirkulare Rippen 5 heraus, deren periphere Begrenzungen durch Rillen 6 unterteilt sind. Erfindungsgemäss sind die Rippen 5 so angeordnet und bemessen, dass bei jeder der beiden Rippen 5 der Aussendurchmesser B der oberen Kanten 9 bzw. 13 kleiner ist als der Innendurchmesser A der Unterkante 10 bzw. 14. Weiterhin besteht zwischen den Rippen 5 die Beziehung, dass der Innendurchmesser D der Oberkante 9 der äquatornäheren Rippe 5 grösser ist als der Aussendurchmesser C der Unterkante 14 der äquatorferneren Rippe 5.

Für die Aufnahme der Rippen 5 sind in den Beckenknochen 15 zusätzlich zu der an die Aussenform des Pfannenkörpers 1 angepassten operativen Ausnehmung zwei Ringnuten 16 (Fig. 3) in den Knochen 15 gefräst.

An der äquatorialen Basis des Pfannenkörpers 1 ist ein vorspringender Rand 8 vorhanden, der einen Winkelbereich von etwa 75° in Umfangsrichtung überdeckt, wobei dieser Bereich relativ zur durch den Zapfen 4 festgelegten Einbaurichtung des Pfannenkörpers 1 so angeordnet ist, dass er einerseits eine Abstützung der Pfanne am Knochen in Richtung der hauptsächlichen Belastungen bewirkt und andererseits die - durch Bewegungen des nicht gezeigten Gelenkkopfes - infolge von Reibung erzeugten Kippmomente auffängt. Der Rand 8 ist daher im wesentlichen im Becken lateral nach aussen gerichtet. Um die in den Körper eingebrachte Fremdmasse gering zu halten, ist der Rand 8 aus einzelnen, durch halbkreisförmige Lücken voneinander getrennten Lappen zusammengesetzt.

Parallel zur Achse 2 kann durch den Pfannenkörper 1 im Scheitel eine nicht dargestellte Bohrung geführt sein, in die ein Gewinde eingeschnitten ist. Dieses dient zum Einschrauben eines geeigneten Setzinstrumentes. Durch die Bohrung erfolgt beim Einschlagen der Pfanne darüberhinaus eine "Entlüftung" des Acetabulums. Im Laufe der Zeit wird die Bohrung durch einwachsendes Gewebe geschlossen.

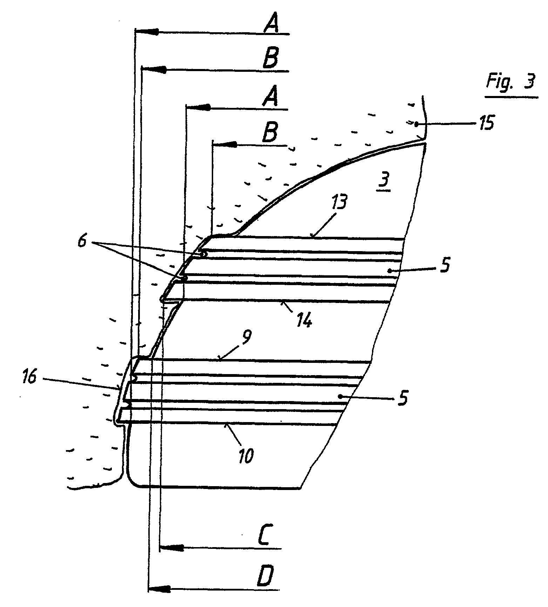

Die neue Pfanne ist vorzugsweise aus Kunststoff hergestellt, insbesondere aus einer der bekannten in der Implantat-Technik verwendeten Polyäthylen-Modifikationen. Die Aussenfläche 3 des Pfannenkörpers 1 kann dabei zusätzlich zu Zapfen 4 und Rippen 5 in bekannter Weise mit einer, das Anwachsen von Gewebe fördernden Oberflächenstruktur 17 (Fig. 2) versehen sein.

**Patentansprüche**

Zementfrei verankerbare Hüftgelenkspfanne mit mindestens annähernd halbkugelförmigem Pfannenkörper (1), dessen äußere Oberfläche (3) im Bereich zwischen 15° und 50° "geographischer Breite" mit zwei Reihen zirkularer Rippen (5) bestückt ist, wobei aus der äußeren Oberfläche (3) ein parallel zur Achse (2) des Pfannenkörpers (1) verlaufender Verankerungszapfen (4) herausragt, und wobei ferner für äquatorparallele Durchmesser (A–D) jeder Rippe (5) gilt, daß der Außendurchmesser (B) der oberen Kante (9, 13) kleiner ist als der Innendurchmesser (A) der Unterkante (10, 14), dadurch gekennzeichnet, daß der Außendurchmesser (C) der Unterkante (14) der äquatorferneren Rippen (5) kleiner ist als der Innendurchmesser (D) der Oberkante (9) der äquatornäheren Rippe (5).

**Claims**

An acetabulum prosthesis adapted to be fixed without cement and having an at least substantially hemispherical socket (1) whose outside surface (3) has two rows of circular ribs (5) in the zone between 15° and 50° of "geographical width", a fixing pin (14) which is parallel to the socket axis (2) extending out of the outside surface (3), it being effective for such diameters (A–D) of each rib (5) as are parallel to the equator that the outer diameter (B) of the top edge (9, 13) is smaller than the inner diameter (A) of the bottom edge (10, 14), characterised in that the outer diameter (C) of the bottom edge (14) of the ribs (5) further away from the equator is smaller than the inner diameter (D) of the top edge (9) of the rib (5) nearer the equator.

**Revendications**

Cavité glénoïde de la hanche à ancrer sans ciment, comportant au moins un corps de cavité (1) à peu près hémisphérique, dont la surface extérieure (3) est pourvue, dans la zone comprise entre 15° et 50° de "latitude", de deux rangées de nervures (5) circulaires, une tige d'ancrage (4) faisant saillie de la surface extérieure (3), parallèlement à l'axe (2) du corps de cavité glénoïde (1), et pour les diamètres parallèles à l'équateur (A–D) de chaque nervure (5), le diamètre extérieur (B) du bord supérieur (9, 13) étant inférieur au diamètre intérieur (A) du bord inférieur (10, 14), caractérisée en ce que le diamètre extérieur (C) du bord inférieur (14) des nervures (5) les plus éloignées de l'équateur est inférieur au diamètre intérieur (D) du bord supérieur (9) de la nervure (5) plus proche de l'équateur.

*Fig. 1*

*Fig. 2*

Fig. 3